# FASCICULE DE BREVET EUROPEEN

(11) **EP 0 993 825 B1**
(45) Date de publication et mention de la délivrance du brevet: **20.06.2001**
(21) Numéro de dépôt: 99401884.4
(22) Date de dépôt: 23.07.1999
(51) Int. Cl.: A61K 7/48, C07D 295/08

(54) **Composition contenant de l'urée et ses utilisations dans le domaine cosmétique et/ou dermatologique**
Harnstoff enthaltende Zusammensetzung und ihre kosmetische und dermatologische Verwendung
Urea containing composition and its cosmetic and dermatologic use

(30) Priorité: 09.09.1998 FR 9811262
(43) Date de publication de la demande: 19.04.2000
(73) Titulaire: L'OREAL, 75008 Paris (FR)
(72) Inventeur: Burnier, Véronique, 75012 Paris (FR)
(74) Mandataire: Rasson, Catherine

(56) Documents cités:
- EP-A- 0 499 229
- DD-A- 281 751
- CHEMICAL ABSTRACTS, vol. 105, no. 4, 28 juillet 1986 (1986-07-28) Columbus, Ohio, US; abstract no. 29780b, C. ACKERMANN ET AL: "Percutaneous absorption of urea" page 363; XP002105546 & INT. J. COSMET. SCI., vol. 7, no. 6, 1985, pages 251-264,
- DATABASE WPI Week 8544 Derwent Publications Ltd., London, GB; AN 85-273407 XP002105548 "Stabilising aq. urea soln. by addn. of taurine" & JP 60 185757 A (SHISEIDO), 21 septembre 1985 (1985-09-21)
- DATABASE WPI Week 8544 Derwent Publications Ltd., London, GB; AN 85-273384 XP002105549 "External application material for skin - contains urea and taurine" & JP 60 185708 A (SHISEIDO), 21 septembre 1985 (1985-09-21)
- DATABASE WPI Week 8838 Derwent Publications Ltd., London, GB; AN 88-267491 XP002105627 "Oil-in-water emulsion stable over wider temp. range - contains aminoalkane sulphonate and/or sulphobetaine amphoteric surfactant" & JP 63 194725 A (LION), 11 août 1988 (1988-08-11)
- DATABASE WPI Week 7624 Derwent Publications Ltd., London, GB; AN 76-44587x XP002105547 "Stabilisation of skin treatment compsn contg urea - using buffer and one or more of ammonium salts, allantoinate and derivs and urea" & JP 51 048441 A (KOWA), 26 avril 1976 (1976-04-26)

## Description

La présente invention a pour objet une composition contenant, de manière stable, de l'urée, destinée en particulier aux domaines cosmétique et/ou dermatologique. L'invention se rapporte aussi à une utilisation de cette composition pour le soin, le traitement, la protection et/ou l'hydratation de la peau humaine, des muqueuses et/ou des fibres kératiniques, ainsi que pour le traitement de la peau sèche.

On utilise couramment dans les compositions cosmétiques ou dermatologiques des humectants, c'est-à-dire des substances hygroscopiques permettant de maintenir la teneur en eau dans la peau et d'influencer ainsi favorablement la souplesse et le toucher de la peau. De tels humectants sont également utiles pour apporter une hydratation à la peau et notamment pour traiter les peaux sèches.

Parmi les nombreux humectants, on peut citer notamment l'urée qui rentre dans la composition du NMF (Natural Moisturizer Factor) et qui a une action amollissante sur les couches cornées de la peau.

Toutefois, l'urée présente l'inconvénient de ne pas être stable en milieu aqueux d'où il se décompose en dioxyde de carbone et en ammoniac. Il s'ensuit une augmentation du pH de la composition le contenant et un dégagement d'odeur qui rendent rédhibitoire l'utilisation de telles compositions.

De ce fait, on cherche depuis longtemps à formuler l'urée dans des compositions cosmétiquement acceptables, sous forme stable pendant au moins plusieurs mois. Ainsi, le document JP51048441 décrit la stabilisation de l'urée dans un tampon glycine-hydroxyde de sodium à un pH de 6 à 9. Toutefois, l'utilisation d'un tel tampon présente l'inconvénient de conférer un effet collant à la composition le contenant lors de l'application sur la peau.

Il subsiste donc le besoin de disposer de compositions stables à base d'urée, ayant des propriétés cosmétiques satisfaisantes.

De manière surprenante et inattendue, la demanderesse a découvert qu'il est possible de formuler des compositions contenant de l'urée, stables dans le temps et agréables à utiliser, en utilisant un acide aminosulfonique N-substitué.

La présente invention a donc pour objet une composition à usage cosmétique et/ou dermatologique, caractérisée par le fait qu'elle contient, dans un milieu physiologiquement acceptable, de l'urée et au moins un acide aminosulfonique N-substitué.

Certes, le document Int. J. Cosmet. Sci., vol.7, n°6, p.251-264 (1985) étudie l'absorption percutanée de l'urée et les causes de l'augmentation du phénomène de perméabilité en relation avec divers composés hydrophiles dont l'acide (N-morpholino)propane sulfonique. Toutefois, ce document ne décrit ni une composition cosmétique ni l'action stabilisante de l'acide (N-morpholino)propane sulfonique.

La composition selon l'invention a l'avantage de pouvoir contenir de l'urée stable après conservation à 45°C pendant 2 mois.

La présente invention a donc aussi pour objet l'utilisation d'au moins un acide aminosulfonique N-substitué pour la stabilisation de l'urée.

La composition selon l'invention a l'avantage d'être particulièrement bien tolérée par la peau et les fibres kératiniques et elle est parfaitement bien adaptée à une utilisation topique et en particulier cosmétique et/ou dermatologique.

L'acide aminosulfonique N-substitué utilisé dans la composition selon l'invention constitue généralement un tampon ayant un pKa allant, à environ 20°C, de 6 à 7,8 et de préférence de 6,3 à 7,6.

L'acide aminosulfonique N-substitué peut être notamment choisi parmi les dérivés aminés des acides alcoylsulfoniques, le groupe alcoyle comportant de préférence de 1 à 6 atomes de carbone et mieux de 2 à 3 atomes de carbone. Il peut s'agir notamment de dérivés aminés de l'acide éthanesulfonique et de l'acide propanesulfonique.

Comme acides aminosulfoniques N-substitués, on peut citer par exemple l'acide N,N-bis[2-hydroxyéthyl]-2-aminoéthanesulfonique (pKa=7,17), l'acide N-2-hydroxyéthyl-piperazine-N'-2-éthanesulfonique (pKa=7,55), l'acide 3-[N-morpholino]-propane-sulfonique (pKa=7,15), l'acide piperazine-N,N'-bis[2-éthanesulfonique] (pKa=6,82), l'acide 3-[N-tris(hydroxyméthyl)méthyl-amino]-2-hydroxypropanesulfonique (pKa=7,6), l'acide 2-[N-morpholino]-éthane-sulfonique (pKa=6,15), l'acide N-(2-acétamido)-2-aminoéthanesulfonique (pKa=6,88), l'acide N-tris(hydroxyméthyl)méthyl-2-aminoéthanesulfonique (pKa=7,5). On peut aussi utiliser un mélange de ces acides. Les pKa indiqués ci-dessus sont ceux déterminés à environ 20°C pour une concentration de 0,1 M.

La quantité d'acide(s) aminosulfonique(s) N-substitué(s) doit être suffisante pour atteindre le résultat escompté, c'est-à-dire la stabilisation de l'urée dans la composition. Cette quantité peut aller par exemple de 0,1 à 20 % et de préférence de 0,5 à 15 % en poids par rapport au poids total de la composition.

La quantité d'urée dans la composition peut varier dans une large mesure selon le but recherché. Pour une application cosmétique et/ou dermatologique, l'urée est présente en une quantité allant généralement de 0,1 à 20 % et de préférence de 0,5 à 15 % en poids par rapport au poids total de la composition.

La composition de l'invention a de préférence un pH d'environ 7 pour assurer une meilleure stabilité de l'urée. On entend par pH d'environ 7, un pH allant de 6,5 à 7,5. Le pH de la composition va de préférence de 6,8 à 7,2. Il peut être ajusté par tout moyen approprié connu et, selon les cas, par un acide inorganique ou organique tel que l'acide chlorhydrique ou l'acide citrique, ou une base inorganique ou organique telle que la soude et la triéthanolamine.

En fonction du pH, l'acide aminosulfonique N-substitué peut se trouver sous forme partiellement salifiée.

Il est connu que l'urée subit une moins forte dégradation quand la composition le contenant est à pH 7. Toutefois, même à ce pH, il se produit une dégradation inexorable dans le temps. Dans la composition de l'invention, la dégradation de l'urée est évitée grâce à la présence de l'acide aminosulfonique N-substitué.

La composition dans laquelle se trouve l'urée comprend un support physiologiquement acceptable, c'est-à-dire compatible avec la peau, les muqueuses y compris les lèvres, et/ou les fibres kératiniques (cheveux, cils). Cette composition peut se présenter sous toutes les formes galéniques à usage topique normalement utilisées et être notamment sous forme de solution aqueuse ou huileuse, de suspension aqueuse ou huileuse ou de dispersion du type lotion ou sérum, d'émulsions obtenues par dispersion d'une phase grasse dans une phase aqueuse (H/E) ou inversement (E/H), d'émulsions triples (E/H/E ou H/E/H) ou de dispersions vésiculaires de type ionique et/ou non ionique. Ces compositions sont préparées selon les méthodes usuelles. Selon une forme préférée de réalisation de l'invention, la composition se présente sous forme d'une émulsion H/E.

Les quantités des différents constituants des compositions sont celles classiquement utilisées dans les domaines considérés.

Lorsque la composition est une émulsion, la proportion de la phase grasse peut aller de 5 à 80 % en poids, et de préférence de 5 à 50 % en poids par rapport au poids total de la composition. Les huiles, les cires, les émulsionnants et les coémulsionnants utilisés dans la composition sous forme d'émulsion sont choisis parmi ceux classiquement utilisés dans les domaines cosmétique et dermatologique. L'émulsionnant et le coémulsionnant sont présents, dans la composition, en une proportion allant de préférence de 0,3 à 30 % en poids, et mieux de 0,5 à 20 % en poids par rapport au poids total de la composition. L'émulsion peut, en outre, contenir des vésicules lipidiques.

Comme huiles ou cires utilisables dans l'invention, on peut citer les huiles minérales (huile de vaseline), les huiles d'origine végétale (fraction liquide du beurre de karité, huile de tournesol), les huiles d'origine animale (perhydrosqualène), les huiles de synthèse (myristate d'isopropyle), les huiles ou cires de silicone (cyclométhicone) et les huiles fluorées (perfluoropolyéthers), les cires d'abeille, de camauba ou paraffine. On peut ajouter à ces huiles des alcools gras (alcool cétylique) et des acides gras (acide stéarique).

Comme émulsionnants utilisables dans l'invention, on peut citer par exemple le stéarate de glycérol et le PEG-50 stéarate.

De façon connue, la composition cosmétique ou dermatologique peut contenir également des adjuvants habituels dans le domaine cosmétique ou dermatologique, tels que les gélifiants hydrophiles ou lipophiles, les additifs hydrophiles ou lipophiles, les conservateurs, les antioxydants, les solvants, les parfums, les charges, les filtres solaires, les absorbeurs d'odeur et les matières colorantes. Les quantités de ces différents adjuvants sont celles classiquement utilisées dans ces domaines, et par exemple de 0,01 à 10 % du poids total de la composition. Ces adjuvants, selon leur nature, peuvent être introduits dans la phase grasse, dans la phase aqueuse et/ou dans les sphérules lipidiques.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium.

Dans la mesure où ils n'interfèrent pas avec l'activité de l'urée, les compositions selon l'invention peuvent contenir d'autres actifs destinés notamment à la prévention et/ou au traitement des affections cutanées.

Comme autres actifs, on peut citer par exemple les polyols tels que la glycérine, les glycols et les dérivés de sucre, les enzymes, les vitamines telles que la vitamine C (acide ascorbique), la vitamine A (rétinol), la vitamine D, la vitamine E (tocophérol), la vitamine K et les dérivés de ces vitamines tels que les esters, les céramides, les dépigmentants tels que l'acide kojique et l'acide caféique, les bêta-hydroxyacides tels que l'acide salicylique et ses dérivés, les alpha-hydroxyacides tels que l'acide lactique et l'acide glycolique, les hydratants tels que les hydrolysats de protéines, les adoucissants tels que l'allantoïne et leurs mélanges.

Les compositions de l'invention sont notamment destinées au soin, au traitement et/ou à la protection de la peau du visage et/ou du corps, et en particulier à l'hydratation de la peau.

Aussi, l'invention a encore pour objet l'utilisation cosmétique de la composition définie ci-dessus pour le soin, le traitement, la protection et/ou l'hydratation de la peau du visage et/ou du corps.

Du fait des propriétés hydratantes de l'urée, la composition de l'invention peut être utilisée pour le traitement de la peau sèche.

L'invention a donc également pour objet l'utilisation de la composition définie ci-dessus pour la préparation d'une composition destinée au traitement de la peau sèche.

Les exemples ci-après permettent d'illustrer l'invention. Dans les compositions, les proportions indiquées sont des pourcentages en poids par rapport au poids total de la composition.

### Exemple 1 : Emulsion H/E

### Phase huileuse :

- Alcool cétylique 7 %
- Stéarate de glycéryle 2,5 %
- PEG-50 stearate 2,5 %
- Huile de vaseline 6,2 %
- Myristate d'isopropyle 3 %

### Phase aqueuse :

- Conservateurs 0.3 %
- Urée 10 %
- Acide 3-[N-morpholino]-propane-sulfonique 7 %
- Soude qsp pH=7 ± 0.2
- Eau qsp 100 %

Mode opératoire : on chauffe la phase huileuse à 80°C. Par ailleurs, on mélange une partie de l'eau de la phase aqueuse et le conservateur et on chauffe le mélange à 80°C, puis on introduit la phase huileuse dans la phase aqueuse sous agitation. On refroidit l'émulsion obtenue à une température d'environ 40°C et on ajoute l'acide 3-[N-morpholino]-propane-sulfonique solubilisé dans un peu d'eau : on ajuste le pH avec de la soude et on introduit dans l'émulsion l'urée solubilisé dans un peu d'eau. On réajuste le pH à environ 7.

Après 2 mois à 45°C, la concentration d'urée dans la composition est restée la même, le pH n'a augmenté que de 0.4 unité, soit une variation de +5.6 %, et la composition stockée ne dégage pas d'odeur, alors que, dans une composition identique sans acide aminosulfonique, la concentration d'urée a diminué de 13 % après 2 mois à 45°C, le pH a augmenté de 1.8 unités, soit une variation de +27 %, et la composition dégage une odeur d'ammoniac.

L'émulsion obtenue a de bonnes propriétés cosmétiques (pas d'effet collant) et est apte à traiter la peau dans le but d'hydrater la peau tout en apportant de la douceur.

### Exemple 2 : Emulsion H/E

### Phase huileuse :

- Alcool cétylique 7 %
- Stéarate de glycéryle 2,5 %
- Stéarate de PEG-50 2,5 %
- Huile de vaseline 6,2 %
- Myristate d'isopropyle 3 %

### Phase aqueuse :

- Urée 10 %
- Acide piperazine-N,N'-bis[2-éthanesulfonique] 10 %
- conservateurs 0,3 %
- Soude qsp pH=7 ± 0.2
- Eau qsp 100 %

Le mode opératoire est le même que pour l'exemple 1.

Après 2 mois à 45°C, la concentration d'urée n'a diminué que de 5,7 %, le pH n'a augmenté que de 0.7 unité, soit une variation de +10 % et la composition ne dégage pas d'odeur, alors que, dans une composition identique sans acide aminosulfonique, la concentration d'urée a diminué de 13 % après 2 mois à 45°C, le pH a augmenté de 1,8 unités, soit une variation de +27 % et la composition dégage une odeur d'ammoniac.

L'émulsion obtenue a de bonnes propriétés cosmétiques (pas d'effet collant) et est apte à traiter la peau dans le but de l'hydrater tout en apportant de la douceur.

### Exemple 3 : Emulsion H/E

### Phase huileuse :

- Alcool cétylique 7 %
- Stéarate de glycéryle 2,5 %
- Stéarate de PEG-50 2,5 %
- Huile de vaseline 6,2 %
- Myristate d'isopropyle 3 %

### Phase aqueuse :

- Urée 10 %
- Acide N,N'-bis[2-Hydroxyéthyl]-2-aminoéthanesulfonique 7.1 %
- conservateurs 0,3 %
- Soude qsp pH=7 ± 0.2
- Eau qsp 100 %

Le mode opératoire est le même que pour l'exemple 1.

Après 2 mois à 45°C, la concentration d'urée n'a diminué que de 7,6 %, le pH n'a augmenté que de 0.7 unité, soit une variation de +10 %, et la composition ne dégage pas d'odeur, alors que, dans une composition identique sans acide aminosulfonique, la concentration d'urée a diminué de 13 % après 2 mois à 45°C, le pH a augmenté de 1,8 unités, soit une variation de +27 % et la composition dégage une odeur d'ammoniac.

L'émulsion obtenue a de bonnes propriétés cosmétiques (pas d'effet collant) et est apte à traiter la peau dans le but de l'hydrater tout en apportant de la douceur.

## Revendications

1. Composition à usage cosmétique et/ou dermatologique, caractérisée par le fait qu'elle contient, dans un milieu physiologiquement acceptable, de l'urée et au moins un acide aminosulfonique N-substitué.

2. Composition selon la revendication 1, caractérisée en ce qu'elle a un pH allant de 6,5 à 7,5.

3. Composition selon la revendication 1 ou 2, caractérisée par le fait que l'acide aminosulfonique N-substitué a un pKa à environ 20°C, de 6 à 7,8.

4. Composition selon l'une quelconque des revendications précédentes, caractérisée par le fait que l'acide aminosulfonique N-substitué est choisi parmi les dérivés aminés des acides alcoylsulfoniques, le groupe alcoyle comportant de 1 à 6 atomes de carbone.

5. Composition selon l'une quelconque des revendications précédentes, caractérisée par le fait que l'acide aminosulfonique N-substitué est choisi parmi l'acide N,N-bis[2-hydroxyéthyl]-2-aminoéthanesulfonique, l'acide N-2-hydroxyéthyl-piperazine-N'-2-éthanesulfonique, l'acide 3-[N-morpholino]-propane-sulfonique, l'acide piperazine-N,N'-bis[2-éthanesulfonique], l'acide 3-[N-tris(hydroxyméthyl)méthyl-amino]-2-hydroxypropanesulfonique, l'acide 2-[N-morpholino]-éthane-sulfonique, l'acide N-(2-acétamido)-2-aminoéthanesulfonique, l'acide N-tris(hydroxyméthyl)méthyl-2-aminoéthanesulfonique et leurs mélanges.

6. Composition selon l'une quelconque des revendications précédentes, caractérisée par le fait que la quantité d'acide(s) aminosulfonique(s) N-substitué(s) va de 0,1 à 20 % en poids par rapport au poids total de la composition.

7. Composition selon l'une quelconque des revendications précédentes, caractérisée par le fait que l'urée est présente en une quantité allant de 0,1 à 20 % en poids par rapport au poids total de la composition.

8. Composition selon l'une quelconque des revendications précédentes, caractérisée par le fait qu'elle se présente sous forme d'une émulsion H/E.

9. Utilisation cosmétique de la composition selon l'une quelconque des revendications précédentes pour le soin, le traitement et/ou la protection de la peau du visage et/ou du corps.

10. Utilisation de la composition selon l'une quelconque des revendications 1 à 8 pour la préparation d'une composition destinée au traitement de la peau sèche.

11. Utilisation d'au moins un acide aminosulfonique N-substitué pour la stabilisation de l'urée.

12. Utilisation selon la revendication 11, caractérisée par le fait que l'acide aminosulfonique N-substitué est choisi parmi les dérivés aminés des acides alcoylsulfoniques, le groupe alcoyle comportant de 1 à 6 atomes de carbone.

## Claims

1. Composition for cosmetic, and/or dermatological use, characterized in that it comprises, in a physiologically acceptable medium, urea and at least one N-substituted aminosulphonic acid.

2. Composition according to Claim 1, characterized in that it has a pH ranging from 6.5 to 7.5.

3. Composition according to Claim 1 or 2, characterized in that the N-substituted aminosulphonic acid has a pKa at approximately 20°C of from 6 to 7.8.

4. Composition according to any one of the preceding claims, characterized in that the N-substituted aminosulphonic acid is selected from amongst the amino derivatives of alkylsulphonic acids, the alkyl group comprising 1 to 6 carbon atoms.

5. Composition according to any one of the preceding claims, characterized in that the N-substituted aminosulphonic acid is selected from amongst N,N-bis[2-hydroxyethyl]-2-aminoethanesulphonic acid, N-2-hydroxyethylpiperazine-N'-2-ethanesulphonic acid, 3-[N-morpholino]propanesulphonic acid, piperazine-N,N'-bis[2-ethanesulphonic] acid, 3-[N-tris-(hydroxymethyl)methylamino]-2-hydroxypropane-sulphonic acid, 2- [N-morpholino]ethanesulphonic acid, N-(2-acetamido)-2-aminoethanesulphonic acid, N-tris-(hydroxymethyl)methyl-2-aminoethanesulphonic acid and mixtures of these.

6. Composition according to any one of the preceding claims, characterized in that the amount of N-substituted aminosulphonic acid(s) ranges from 0.1 to 20% by weight, based on the total weight of the composition.

7. Composition according to any one of the preceding claims, characterized in that the urea is present in a quantity ranging from 0.1 to 20% by weight, based on the total weight of the composition.

8. Composition according to any one of the preceding claims, characterized in that it is in the form of an O/W emulsion.

9. Cosmetic use of the composition according to any one of the preceding claims for the care, treatment and/or protection of the skin of the face and/or the body.

10. Use of the composition according to any one of Claims 1 to 8 for the preparation of a composition intended for the treatment of dry skin.

11. Use of at least one N-substituted aminosulphonic acid for the stabilization of urea.

12. Use according to Claim 11, characterized in that the N-substituted aminosulphonic acid is selected from amongst the amino derivatives of alkylsulphonic acids, the alkyl group comprising 1 to 6 carbon atoms.

## Patentansprüche

1. Zusammensetzung zur kosmetischen und/oder dermatologischen Anwendung, dadurch gekennzeichnet, dass sie in einem physiologisch akzeptablen Medium Harnstoff und mindestens eine N-substituierte Aminosulfonsäure enthält.

2. Zusammensetzung nach Anspruch 1, dadurch gekennzeichnet, dass sie einen pH-Wert im Bereich von 6,5 bis 7,5 aufweist.

3. Zusammensetzung nach Anspruch 1 oder 2, dadurch gekennzeichnet, dass die N-substituierte Aminosulfonsäure bei etwa 20 °C einen pKa-Wert von 6 bis 7,8 aufweist.

4. Zusammensetzung nach einem der vorhergehenden Ansprüche, dadurch gekennzeichnet, dass die N-substituierte Aminosulfonsäure unter aminierten Derivaten von Alkylsulfonsäuren ausgewählt ist, wobei die Alkylgruppe 1 bis 6 Kohlenstoffatome aufweist.

5. Zusammensetzung nach einem der vorhergehenden Ansprüche, dadurch gekennzeichnet, dass die N-substituierte Aminosulfonsäure ausgewählt ist unter N,N-Bis[2-hydroxyethyl]-2-aminoethansulfonsäure, N-2-Hydroxyethyl-piperazin-N'-2-ethansulfonsäure, 3-[N-Morpholino]-propansulfonsäure, Piperazin-N,N'-bis[2-ethansulfonsäure], 3-[N-Tris(hydroxymethyl)methyl-amino]-2-hydroxypropansulfonsäure, 2-[N-Morpholino]-ethansulfonsäure, N-(2-Acetamido)-2-aminoethansulfonsäure und N-Tris(hydroxymethyl)methyl-2-aminoethansulfonsäure und den Gemischen dieser Verbindungen.

6. Zusammensetzung nach einem der vorhergehenden Ansprüche, dadurch gekennzeichnet, dass der Mengenanteil der N-substituierten Aminosulfonsäure(n) im Bereich von 0,1 bis 20 Gew.-%, bezogen auf das Gesamtgewicht der Zusammensetzung, liegt.

7. Zusammensetzung nach einem der vorhergehenden Ansprüche, dadurch gekennzeichnet, dass der Harnstoff in einem Mengenanteil von 0,1 bis 20 Gew.-%, bezogen auf das Gesamtgewicht der Zusammensetzung, vorliegt.

8. Zusammensetzung nach einem der vorhergehenden Ansprüche, dadurch gekennzeichnet, dass sie in Form einer O/W-Emulsion vorliegt.

9. Kosmetische Verwendung der Zusammensetzung nach einem der vorhergehenden Ansprüche zur Pflege, zur Behandlung und/oder zum Schutz der Gesichtshaut und/oder der Körperhaut.

10. Verwendung der Zusammensetzung nach einem der Ansprüche 1 bis 8 zur Herstellung einer Zusammensetzung, die zur Behandlung von trockener Haut dienen soll.

11. Verwendung mindestens einer N-substituierten Aminosulfonsäure zur Stabilisierung von Harnstoff.

12. Verwendung nach Anspruch 11, dadurch gekennzeichnet, dass die N-substituierte Aminosulfonsäure unter aminierten Derivaten von Alkylsulfonsäuren ausgewählt ist, wobei die Alkylgruppe 1 bis 6 Kohlenstoffatome aufweist.
